(19) Europäisches Patentamt
European Patent Office
Office européen des brevets

(11) Veröffentlichungsnummer: **0 298 399**
**A1**

# (12) EUROPÄISCHE PATENTANMELDUNG

(21) Anmeldenummer: **88110610.8**

(22) Anmeldetag: **02.07.88**

(51) Int. Cl.⁴: **C07D 411/04 , C07D 307/62 , C09K 15/12 , A23L 3/34**

Patentansprüche für folgenden Vertragsstaat: ES.

(30) Priorität: **09.07.87 CH 2616/87**
**25.04.88 CH 1536/88**

(43) Veröffentlichungstag der Anmeldung:
**11.01.89 Patentblatt 89/02**

(84) Benannte Vertragsstaaten:
**AT BE CH DE ES FR GB GR IT LI NL**

(71) Anmelder: **F. HOFFMANN-LA ROCHE & CO. Aktiengesellschaft**

**CH-4002 Basel(CH)**

(72) Erfinder: **Pauling, Horst, Dr.**
**Ruchholzstrasse 39**
**CH-4103 Bottmingen(CH)**
Erfinder: **Wehrli, Christof**
**In den Gärten 1**
**CH-4108 Witterswil(CH)**

(74) Vertreter: **Urech, Peter, Dr. et al**
**Grenzacherstrasse 124 Postfach 3255**
**CH-4002 Basel(CH)**

## (54) Antioxydantien und deren Herstellung.

(57) Es werden die neue 5,6-Sulfinyl-L-ascorbinsäure und deren Stereoisomere, ihre Herstellung durch Sulfinylierung von L-Ascorbinsäure oder vom entsprechenden Stereoisomeren, Kompositionen mit oxydationshemmenden Eigenschaften, die durch einen Gehalt an 5,6-Sulfinyl-L-ascorbinsäure oder einem Stereoisomeren davon gekennzeichnet sind, sowie die Verwendung der 5,6-Sulfinyl-L-ascorbinsäure oder eines Stereoisomeren davon als Antioxydans beschrieben.

EP 0 298 399 A1

Xerox Copy Centre

## Antioxydantien und deren Herstellung

Die Erfindung betrifft neue Antioxydantien, nämlich die 5,6-Sulfinyl-L-ascorbinsäure, also die Verbindung der Formel

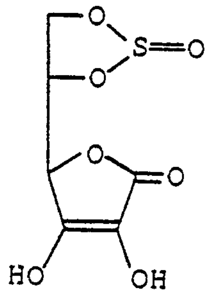

I

und deren Stereoisomere, also die Verbindungen der Formeln

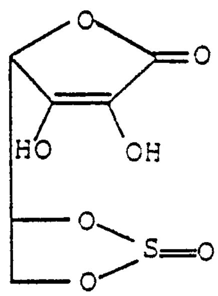

II

(5,6-Sulfinyl-D-ascorbinsäure)

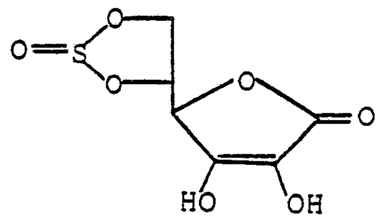

III

(5,6-Sulfinyl-D-erythorbinsäure)

und

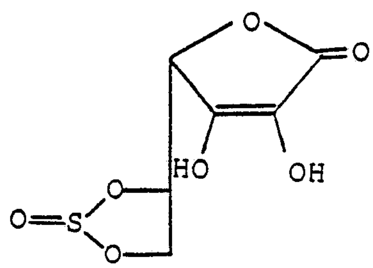

IV

(5,6-Sulfinyl-L-erythorbinsäure)

Die Erfindung betrifft auch ein Verfahren zur Herstellung von 5,6-Sulfinyl-L-ascorbinsäure und von deren Stereoisomeren. Dieses Verfahren ist dadurch gekennzeichnet dass man L-Ascorbinsäure oder das entsprechende Stereoisomere, also D-Ascorbinsäure, D-Erythorbinsaure bzw. L-Erythorbinsäure, sulfinyliert.

Die Erfindung betrifft schliesslich die Verwendung der Verbindungen I bis IV als Antioxydantien sowie Kompositionen mit oxydationshemmenden Eigenschaften, die durch einen Gehalt an 5,6-Sulfinyl-L-ascorbinsäure oder einem Stereoisomeren davon gekennzeichnet sind.

Die Sulfinylierung der L-Ascorbinsäure bzw. ihrer Stereoisomeren erfolgt zweckmässigerweise unter Verwendung von Thionylchlorid als Sulfinylierungsmittel. Die Reaktionspartner werden zweckmässigerweise in ungefähr äquimolekularen Mengen, vorzugsweise in möglichst äquimolaren Mengen, zur Reaktion gebracht. Zudem erfolgt die Sulfinylierung zweckmässigerweise bei Temperaturen von ca. -20° C bis ca. 50° C. Ein bevorzugter Bereich ist derjenige von ca. -10° C bis ca. 25° C.

Man arbeitet zweckmässigerweise unter Zuhilfenahme eines inerten Lösungsmittels, insbesondere eines polaren, aprotischen Lösungsmittels; Beispiele solcher Lösungsmittel sind acyclische Di- und Polyäther, wie Monoglyme (1,2-Dimethoxyäthan) und Diäthylenglykoldimethyläther; cyclische Mono- und Diäther, wie Tetrahydrofuran und Dioxan; Sulfolan (Tetrahydrothiophen-1,1-dioxid); Dimethylformamid; Dimethylacetamid; N-Methylpyrrolidon; und Acetonitril.

Die neuen Verbindungen besitzen interessante oxydationshemmende Eigenschaften und können in einer breiten Palette von Substraten eingesetzte werden z.B.:

- in Lebensmitteln [Wein (insbesondere für dessen Schwefelung), Speiseessig, Speiseöle und -fette, Bier, Frucht- und Gemüsesäfte. Sirup, Fleisch und Wurstwaren, Dörrobst, Trockengemüse, Nüsse, Obst- und Gemüsekonserven, Kartoffelprodukte, Brotaufstriche, Saucen, Mayonnaise, etc.]
- in Futtermitteln
- in Kesselspeisewasser
- in Mitteln zur Herstellung und Entwicklung von Filmen
- in Druckmitteln
- in Mitteln zur Herstellung und Behandlung von Textilien
- in Kunststoffen
- in Schmierölen und -fetten
- in Kautschuk

Die zweckmässig eingesetzte Menge der Verbindung I, II, III oder IV kann jeweils klein gehalten werden; sie entspricht vorzugsweise ungefähr der eingesetzten Menge anderer Antioxydantien des Sulfit-typs,

beträgt also z.B. zweckmässigerweise etwa ca. 1-7000 ppm. insbesondere ca. 20 bis ca. 2000 ppm. Die bevorzugte Menge richtet sich nach dem Anwendungszweck. In der Lebensmittelindustrie beträgt diese bevorzugte Menge beispielsweise:

für Wein: ca. 50-300 ppm
für Dörrfrüchte: ca. 1000-7000 pm
für Frucht- und Gemüsesäfte: ca. 80-300 ppm

Die neuen Verbindungen I bis IV können als solche dem zu schützenden Substrat zugegeben werden wo zweckdienlich, aber auch verdünnt, z.B. als Lösung, insbesondere als wässrige oder als alkoholische Lösung.

Bei höheren Konzentrationen (≧ 2 mg/ml Substrat) wirken die Verbindungen I bis IV zudem bakteriologisch und fungizid.

Beispiel

In einen mit Thermometer und Rührer ausgestatteten 350 ml Vierhalssulfierkolben werden unter Schutzgasatmosphäre ($N_2$) 17,6 g (100 mMol) kristalline L-Ascorbinsäure und 50 ml Tetrahydrofuran vorgelegt. Dazu werden bei 0 °C 7,42 ml (102 mMol) Thionylchlorid zugegeben, und das Ganze wird 24 Stunden bei 0 °C gerührt. Nach dieser Zeit hat sich die Ascorbinsäure praktisch vollständig gelöst. Die Lösung wird auf 200 g Eis/Wasser gegossen und in 4 Scheidetrichtern mit 4x200 ml Essigester extrahiert. Die organischen Phasen werden mit 8x50 ml 20%iger Kochsalzlösung gewaschen. Die Essigesterphasen werden vereinigt, über wasserfreiem Natriumsulfat getrocknet und am Rollverdampfer im Wasserstrahlvakuum bis zur Trockene eingeengt. Der Rückstand (21,2 g) wird in 45 ml Essigester gelöst. Ein geringer unlöslicher Anteil wird abgenutscht und mit 5 ml Essigester nachgewaschen. Zum Filtrat werden langsam unter Rühren 100 ml Toluol zugetropft. Nach Animpfen wird 18 Stunden gerührt. Das Produkt wird genutscht und 8 Stunden bei 45 °C im Wasserstrahlvakuum bis zur Gewichtskonstanz getrocknet. Man erhält 17,6 g 5,6-Sulfinyl-L-ascorbinsäure (79% der theoretischen Ausbeute): Schmelzpunkt 150-151 °C (Zersetzung); $[\alpha]^{20}_{365}$ = +246,2° (1% in $H_2O$).

| Elementaranalyse: | C% | H% | S% |
|---|---|---|---|
| ber. | 32,44 | 2,72 | 14,43 |
| gef. | 32,68 | 2,66 | 14,00 |

Analog verläuft unter Verwendung von D-Ascorbinsäure D-Erythorbinsäure oder L-Erythorbinsäure die Synthese des entsprechenden 5,6-sulfinylierten Derivats.

## Ansprüche

1. 5,6-Sulfinyl-L-ascorbinsäure und deren Stereoisomere.
2. 5,6-Sulfinyl-L-ascorbinsäure.
3. Verfahren zur Herstellung von 5,6-Sulfinyl-L-ascorbinsäure und von deren Stereoisomeren, dadurch gekennzeichnet, dass man L-Ascorbinsäure oder das entsprechende Stereoisomere sulfinyliert.
4. Verfahren nach Anspruch 3, dadurch gekennzeichnet, dass man L-Ascorbinsäure zur 5,6-Sulfinyl-L-ascorbinsaure sulfinyliert.
5. Verfahren nach Anspruch 3 oder 4, dadurch gekennzeichnet, dass man mittels Thionylchlorid sulfinyliert.
6. Komposition mit oxydationshemmenden Eigenschaften gekennzeichnet durch einen Gehalt an 5,6-Sulfinyl-L-ascorbinsäure oder einem Stereoisomeren davon.
7. Komposition nach Anspruch 6, gekennzeichnet durch einen Gehalt an 5,6-Sulfinyl-L-ascorbinsäure.
8. Verwendung von 5,6-Sulfinyl-L-ascorbinsäure oder eines Stereoisomeren davon als Antioxydans.
9. Verwendung von 5,6-Sulfinyl-L-ascorbinsäure als Antioxydans.

Patentansprüche für folgende Vertragsstaaten: ES

1. Komposition mit oxydationshemmenden Eigenschaften. gekennzeichnet durch einen Gehalt an 5,6-Sulfinyl-L--ascorbinsäure oder einem Stereoisomeren davon.

2. Komposition nach Anspruch 1, gekennzeichnet durch einen Gehalt an 5,6-Sulfinyl-L-ascorbinsäure.

3 Verfahren zur Herstellung von 5,6-Sulfinyl-L--ascorbinsäure und von deren Stereoisomeren, dadurch gekennzeichnet. dass man L-Ascorbinsäure oder das entsprechende Stereoisomere sulfinyliert.

4. Verfahren nach Anspruch 3, dadurch gekennzeichnet, dass man L-Ascorbinsäure zur 5,6-Sulfinyl-L-ascorbinsäure sulfinyliert.

5. Verfahren nach Anspruch 3 oder 4, dadurch gekennzeichnet. dass man mittels Thionylchlorid sulfinyliert.

6. Verfahren zur Herstellung einer Komposition mit oxydationshemmenden Eigenschaften, dadurch gekennzeichnet, dass man 5,6-Sulfinyl-L-ascorbinsäure oder ein Stereoisomeres davon mit dem gewünschten Substrat vermischt.

7. Verfahren nach Anspruch 6, dadurch gekennzeichnet, dass man 5,6-Sulfinyl-L-ascorbinsäure mit dem gewünschten Substrat vermischt.

8. Verwendung von 5,6-Sulfinyl-L-ascorbinsäure oder eines Stereoisomeren davon als Antioxydans.

9. Verwendung von 5,6-sulfinyl-L-ascorbinsäure als Antioxydans.

Europäisches Patentamt

# EUROPÄISCHER RECHERCHENBERICHT

Nummer der Anmeldung

EP 88 11 0610

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CHEMICAL ABSTRACTS, Band 74, Nr. 17, 26. April 1971, Seiten 465-466, Abstract-Nr. 88266c, Columbus, Ohio, USA; & JP - A - 70 31661 | 1,6-9 | C 07 D 411/04<br>C 07 D 307/62<br>C 09 K 15/12<br>A 23 L 3/34 |
| A | EP-A-0 146 121 (TAKEDA CHEMICAL INDUSTRIES LTD.)<br>* Ansprüche 1,10 * | 1,6-9 | |
| A | PATENT ABSTRACTS OF JAPAN, Band 11, Nr. 80 (C-409)[2527], 11. März 1987; & JP - A - 61 236 772 (TAKEDA CHEMICAL INDUSTRIES LTD.) 22.10.1986 | 1,6-9 | |
| A | PATENT ABSTRACTS OF JAPAN, Band 11, Nr. 69 (C-407)[2516], 3. März 1987; & JP - A - 61 227 578 (NEOS CO. LTD.) 09.10.1986 | 1-5 | |
| A | CHEMICAL ABSTRACTS, Band 92, Nr. 19, 12. Mai 1980, Seite 267, Abstract-Nr. 164180j; Y. WANG et al.: "The synthesis and the proton and carbon -13 nuclear magnetic resonance spectroscopy of the cyclic sulfites of some sugars"; & CARBOHYDR. RES., 1979, 76, 131-40 | 1-5 | **RECHERCHIERTE SACHGEBIETE (Int. Cl.4)**<br>C 07 D 307/00<br>C 07 D 327/00<br>C 07 D 411/00 |
| A | CHEMICAL ABSTRACTS, Band 102, Nr. 13, 1. April 1985, Seite 751, Abstract-Nr. 113861z; V.A. TIMOSHCHUK: "Synthesis of N1-(3-chloro-3-deoxy-2,4,6-tri-O-=acetyl -alpha-D-allopyranosyl)-5-fluorouracil" * compound I *; & ZH. OBSHCH. KHIM. 1984, 54 (11), 2646-7 | 1,2 | |

Der vorliegende Recherchenbericht wurde für alle Patentansprüche erstellt

| Recherchenort | Abschlußdatum der Recherche | Prüfer |
|---|---|---|
| BERLIN | 03-10-1988 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)